(19) Europäisches Patentamt
European Patent Office
Office européen des brevets

(11) Publication number: **0 370 563 B1**

# (12) EUROPEAN PATENT SPECIFICATION

(45) Date of publication of patent specification: **19.05.93** (51) Int. Cl.5: **C07C 11/12**, C07C 2/74

(21) Application number: **89202888.7**

(22) Date of filing: **14.11.89**

## (54) Preparation of conjugated dienes.

(30) Priority: **23.11.88 GB 8827303**

(43) Date of publication of application:
**30.05.90 Bulletin 90/22**

(45) Publication of the grant of the patent:
**19.05.93 Bulletin 93/20**

(84) Designated Contracting States:
**AT BE CH DE ES FR GB IT LI NL SE**

(56) References cited:
**EP-A- 0 012 472**
**EP-A- 0 012 475**
**EP-A- 0 019 960**
**EP-A- 0 019 961**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Goodall, Brian Leslie**
**3959 Clover Hill Road**
**Akron Ohio(US)**
Inventor: **Terlouw, Willem**
**Badhuisweg 3**
**NL-1031 CM Amsterdam(NL)**
Inventor: **Van der Sar, Jacob Cornelius**
**Badhuisweg 3**
**NL-1031 CM Amsterdam(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to a process for the preparation of certain conjugated dienes. In particular it relates to a process for preparing 2,3-dimethyl-butadiene, (E)-3-methyl-4-methylene-2-hexene and (E,E)-3,4-dimethyl-2,4-hexadiene.

Conjugated dienes are useful as monomers in the preparation of a wide variety of polymers such as elastomers and rubbers, and as chemical intermediates, for example in the preparation of pharmaceuticals and agrochemicals. Thus, for example, United States patent number 4532301 describes the use of 2,3-dimethylbutadiene in the preparation of radial block polymers.

European patent number 0 004 409 describes a process for the linear hydrodimerization of 1,3-butadiene in which 1,3-butadiene is contacted with a catalytic amount of palladium or a palladium compound, a tertiary phosphine, formic acid and a base which can neutralize formic acid, optionally in the presence of a solvent to yield 1,7-octadiene. There is no mention of the hydrodimerization of a butadiene to yield a conjugated diene.

It has now been found that 2,3-dimethylbutadiene, (E)-3-methyl-4-methylene-2-hexene and (E,E)-3,4-dimethyl-2,4-hexadiene can advantageously be prepared by hydrodimerising propadiene or 1,2-butadiene.

Accordingly the present invention provides a process for the preparation of a compound of general formula

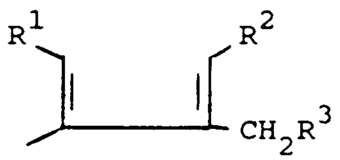 (I)

in which $R^1$ represents a hydrogen atom or a methyl group, one of $R^2$ and $R^3$ represents a hydrogen atom and the other of $R^2$ and $R^3$ is the same as $R^1$, which comprises reacting a compound of general formula

$R^1CH=C=CH_2$ (II)

with a salt of formic acid in the presence of a catalyst system comprising a palladium salt and a phosphorus compound of general formula

$P(OX)_n(X)_{3-n}$ (III)

in which n is 0,1,2 or 3 and each X independently represents an alkyl, alkenyl, aryl, aralkyl, alkaryl or cycloalkyl group, and recovering the desired compound of formula (I).

The process according to the invention affords the compounds of general formula (I) in high yield with good conversion. This is both surprising and advantageous. Thus it is surprising that the process does not afford substantial amounts of polymeric materials and isomers of the compounds of formula (I).

Both propadiene and 1,2-butadiene are cheap and readily available commercial starting materials. An important and most surprising advantage of the process of this invention is that propadiene can be selectively hydrodimerized to yield 2,3-dimethyl butadiene from a mixed stream containing propadiene and other $C_3$ compounds, notably propyne. Accordingly, it follows that the starting materials for the process of this invention, propadiene and 1,2-butadiene, may be present as pure compounds or, particularly in the case of propadiene, as a mixture of compounds.

In the process according to the invention the salt of formic acid may optionally be generated in situ preferably by reacting formic acid with an appropriate base. When the salt of formic acid is generated in situ, it is preferable that a sufficient amount of base is used to at least neutralise the formic acid present in the reactor. The formic acid may be added at the start of the reaction and/or continuously or stepwise during the reaction. An advantage of adding the formic acid continuously or stepwise is that less base is required. It has also been observed that continuous addition of formic acid in the hydrodimerisation of 1,2-butadiene increases the selectivity of the reaction in favour of (E)-3-methyl-4-methylene-2-hexene.

Preferred salts of formic acid are alkali metal salts, for example those formed by reaction with alkali metal hydroxides or carbonates such as sodium or potassium hydroxide or carbonate, and salts of tertiary organic amines, for example triethylamine.

The palladium salt employed in the process according to the invention is conveniently a carboxylate, nitrate or halide. Palladium acetate is particularly preferred.

The molar ratio of the compound of formula (II) to palladium salt is not critical, and will be determined primarily by economic and practical considerations. It is preferably not more than 50,000.

Referring to the phosphorus compound of general formula (III), any alkyl or alkenyl group present preferably has from 1 to 20, more preferably from 3 to 10 carbon atoms. Preferably any branching occurs at a carbon atom no more than two carbon atoms from the phosphorus atom, as for example in an isopropyl group. An aryl group is preferably a phenyl group. An aralkyl group is preferably a benzyl group. An alkaryl group is preferably a ditertiarybutyl phenyl group or an ortho-tolyl group. A cycloalkyl group preferably has from 3 to 6 carbon atoms.

In general, particularly preferred phosphorus compounds of general formula (II) are triphenylphosphine, tri-isopropylphosphine, diphenylisopropylphosphine, tribenzylphosphine, tri(ortho-toluyl)phosphine and tri(2,4-di-tertiarybutylphenyl)phosphite. Optionally more than one phosphorus compound may be employed. For example a mixture of triphenylphosphine and tri-(2,4-di-tertiarybutylphenyl)phosphite may be used. However, the use of tri-isopropylphosphine alone is most preferred.

When using as starting material a mixture of propadiene and other $C_3$ compounds, most notably propyne, preferred phosphorus compounds of general formula(II) are those in which n is O and X represents an alkyl group. The alkyl group represented by X is preferably a branched chain alkyl group, isopropyl amd isobutyl being especially preferred. The use of tri-isopropylphosphine as the phosphorus compound is most preferred.

The molar ratio of phosphorus compound of general formula (III) to palladium salt is conveniently in the range of from 1:1 to 20:1, preferably 1.5:1 to 4:1.

The process according to the invention is conveniently effected at a temperature in the range of from 30 to 100 °C, preferably from 40 to 70 °C.

A solvent is not essential in the process according to the invention. However, the reaction is preferably performed in the presence of a solvent. Suitable solvents include amides such as dimethylformamide or dimethylacetamide; tertiary amines such as triethylamine or pyridine; aromatic hydrocarbons such as benzene, toluene or the three xylenes; sulphoxides such as dimethylsulphoxide; ethers such as tetrahydrofuran; nitriles such as benzonitrile; nitro compounds such as nitromethane or nitrobenzene; halogenated hydrocarbons such as chlorobenzene and chloroform; and carbonates such as propylene carbonate.

Preferably the reaction is performed in the presence of a polar aprotic solvent.

The process is conveniently effected at a pressure in the range of from 2 to 25 bar, most preferably under autogenous pressure.

The compounds of general formula (I) may be recovered from the reaction mixture using conventional techniques such as distillation. Thus as described in J. Am. Chem. Soc., Vol 110, No. 6, 1988, 1883-1889, (E)-3-methyl-4-methylene-2-hexene has a boiling point of 127 °C at 760mm Hg, and (E,E)-3,4-dimethyl-2,4-hexadiene has a boiling point of 134.5 °C at 760mm Hg.

The following Examples illustrate the invention.

Example 1

Preparation of 2,3-dimethylbutadiene

A 250ml stainless steel reactor (flushed with nitrogen) was loaded with dimethylformamide (40ml) and a solution (in a further 10ml of dimethylformamide) of a catalyst consisting of palladium acetate (0.1mmol) and triisopropylphosphine (0.2mmol). To this homogeneous system was added a mixture of triethylamine (0.4mol) and formic acid (0.4mol). Finally propadiene (1mol, 25% excess on the basis of formic acid) was added and the temperature was raised from 20 °C to 40 °C. After 24 hours, the conversion, based on formic acid, was 100% and only one product, 2,3-dimethylbutadiene, was obtained.

Example 2

Preparation of 2,3-dimethylbutadiene

The method of Example 1 was repeated except that triphenylphosphine was used instead of triisopropylphospine, the temperature was raised to 40 °C and the duration of the reaction was 23 hours. 100% conversion, based on formic acid was observed yielding one product, 2,3-dimethylbutadiene.

Examples 3 and 4

The method of Example 1 was repeated except that a mixture of propadiene and propyne, in the proportions 75% to 25% respectively, was used as starting material instead of propadiene. The method of Example 1 was again repeated except that propyne was used as starting material instead of propadiene. The amounts of reactants, the reaction conditions and the results of these two experiments are summarized in Table 1.

Table 1

| Ex. No. | Propadiene mol | Propyne mol | Formic acid mol | base mol | Pd $(OAc)_2$ mmol | Phosphorus compound mmol | Solvent ml | Temp °C | Time hours | Conversion % | Selectivity 2,3-DMBD |
|---|---|---|---|---|---|---|---|---|---|---|---|
| 3 | 0.75 | 0.25 | 0.4 | $Et_3N$ 0.4 | 0.1 | $P(iPr)_3$ 0.2 | 100 | 40 | 19 | 33 | 100 |
| 4 | 0 | 1.0 | 0.4 | $Et_3N$ 0.4 | 0.1 | $P(iPr)_3$ 0.2 | 100 | 40 | 20 | 0 | - |

2,3 DMBD: 2,3-dimethylbutadiene

i-Pr : isopropyl

Example 5

Preparation of (E)-3-methyl-4-methylene-2-hexene and (E,E)-3,4-dimethyl-2,4-hexadiene

To dimethylformamide (100ml) in a 250ml stainless steel reactor (flushed with nitrogen) was added formic acid (9.2g, 0.2mol); triethylamine (20.2g, 0.2mol); palladium acetate (22mg, 0.1 mmol), triphenylphosphine (60mg, 0.2 mmol) and 1,2-butadiene (22g, 0.4mol). The reaction mixture was heated to 50°C with stirring for 2.5 hours, after which time the reaction was stopped. 81% conversion, based on formic acid, was observed with the following product distribution:- (E)-3-methyl-4-methylene-2-hexene (65%); (E,E)-3,4-dimethyl-2,4-hexadiene (27%) and 1,7-octadiene (8%). The products are readily separable by distillation.

Example 6

Preparation of (E)-3-methyl-4-methylene-2-hexene and (E,E)-3,4-dimethyl-2,4-hexadiene

The method of Example 5 was repeated, except that triisopropylphosphine was used instead of triphenylphosphine, the reaction mixture was heated to 60°C, and the reaction was stopped after 22 hours. 50% conversion, based on formic acid, was observed with the following product distribution: (E)-3-methyl-4-methylene-2-hexene (47%); (E,E)-3,4-dimethyl-2,4-hexadiene (46%) and 1,7-octadiene (7%).

Examples 7 to 16

Ten further experiments were performed using 1,2-butadiene as the substrate following methods analogous to those described in Examples 1 to 6. In each case the palladium salt was palladium acetate. In Examples 5 to 13 the solvent was dimethylformamide (DMF), while in Example 14 it was propylene carbonate. As with Examples 1 to 6, the pressure was autogenous (typically starting at 3 bar and rising to about 7 bar during the reaction). The base and formic acid were always mixed together in the solvent before the catalyst components (phosphorus compound and palladium acetate dissolved in solvent) and 1,2-butadiene were added.

The amounts of reactants, the reaction conditions and the results are summarised in Table 2. In each case, the percentage conversion is based on formic acid.

Example 17

A 6 1 stainless steel reactor (under nitrogen) was loaded with dimethyl formamide (1000 ml), triethylamine (4 mol) and formic acid (3.8 mol), and a clear solution of palladium acetate (1 mmol) and tri-isopropylphosphine (1.9 mmol) in 50 ml additional dimethylformamide, and 1,2-butadiene (8.3 mol) were added. The reaction mixture was heated to 43-48°C with stirring for 25 hours, after which the reaction was complete. The product distribution was: (E)-3-methyl-4-methylene-2-hexene (53%), (E,E)-3,4-dimethyl-2,4-hexadiene (27%) and 1,7-octadiene (6%).

Example 18

A 6 1 stainless steel reactor (under nitrogen) was loaded with a clear solution of palladium acetate (3.0 mmol), tri-isopropylphosphine (6.0 mmol) in triethylamine (3.0 mol) and 1,2-butadiene (15.6 mol). During heating the reactor to 40-45°C, formic acid (1.5 mol) was added in one portion. After three hours reaction time, formic acid (6.0 mol) was added continuously over 11 hours with stirring at reaction temperature and the reaction was allowed to complete in a further 10 hours. The product distribution was: (E)-3-methyl-4-methylene-2-hexene (76%); (E,E)-3,4-dimethyl-2,4-hexadiene (19%) and 1,7-octadiene (4%).

Table 2

| Ex. No. | 1,2-butadiene mol | formic acid mol | base mol | Pd (OAc)$_2$ mmol | Phosphorus compound mmol | Solvent | Temp °C | Time hours | Conversion % | Selectivity | | |
|---|---|---|---|---|---|---|---|---|---|---|---|---|
| | | | | | | | | | | MMH* %w | DMHXD* %w | OD* %w |
| 7 | 0.22 | 0.1 | KOH 0.1 | 0.1 | $P(iPr)_3$ 0.2 | 100ml | 70 | 18 | 40 | 53 | 37 | 10 |
| 8 | 0.4 | 0.2 | KOH 0.2 | 0.1 | $PPh_3$ 0.3 | 100ml | 50 | 12 | 28 | 65 | 28 | 7 |
| 9 | 0.4 | 0.2 | $Et_3N$ 0.2 | 0.1 | $PPh_3$ 3.6 | 100ml | 95 | 0.5 | 72 | 64 | 28 | 8 |
| 10 | 0.4 | 0.2 | $Na_2CO_3$ 0.1 | 0.1 | DTBP 0.3 | 100ml | 50 | 48 | 22 | 45 | 34 | 21 |

Table 2 (cont'd)

| Ex. No. | 1,2-butadiene mol | Formic acid mol | base mol | Pd $(OAc)_2$ mmol | Phosphorus compound mmol | Solvent | Temp °C | Time hours | Conversion % | Selectivity MMH* %w | Selectivity DMHXD* %w | Selectivity OD* %w |
|---|---|---|---|---|---|---|---|---|---|---|---|---|
| 11 | 0.4 | 0.2 | $Et_3N$ 0.2 | 0.1 | DTPB 1.0 | 100ml | 50 | 6 | 25 | 60 | 33 | 7 |
| 12 | 0.4 | 0.2 | $Et_3N$ 0.2 | 0.1 | DTPB 0.2 +$PPh_3$ 0.1 | 100ml | 50 | 6 | 93 | 60 | 33 | 7 |
| 13 | 0.8 | 0.38 | $Et_3N$ 0.4 | 0.1 | $PPh_3$ 0.2 | 100ml | 42 | 21 | 100 | 67 | 27 | 6 |
| 14 | 1.2 | 0.38 | $Et_3N$ 0.4 | 0.1 | $PPh_2$(i-Pr) 0.2 | 100ml | 42 | 20 | 100 | 71 | 25 | 4 |

Table 2 (cont'd)

| Ex. No. | 1,2-butadiene mol | Formic acid mol | base mol | Pd $(OAc)_2$ mmol | Phosphorus compound mmol | Solvent | Temp °C | Time hours | Conversion % | Selectivity MMH* %w | DMHXD* %w | OD* %w |
|---|---|---|---|---|---|---|---|---|---|---|---|---|
| 15 | 1.0 | 0.38 | $Et_3N$ 0.4 | 0.1 | $P(i\text{-}Pr)_3$ 0.2 | 100ml | 42 | 20 | 97 | 55 | 41 | 4 |
| 16 | 0.8 | 0.38 | $Et_3N$ | 0.1 | $PPh_3$ | 114g | 42 | 20 | 100 | 60 | 35 | 5 |

MMH : (E)-3-methyl-4-methylene-2-hexene
DMHXD : (E,E)-3,4-dimethyl-2,4-hexadiene
OD : 1,7-octadiene
i-Pr : isopropyl
Ph : phenyl
DTPB : tri(2,4-di-tertiarybutylphenyl)phosphite

## Claims

**1.** A process for the preparation of a compound of general formula

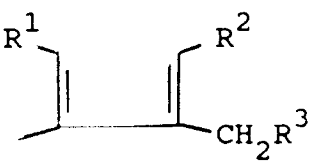

(I)

in which $R^1$ represents a hydrogen atom or a methyl group, one of $R^2$ and $R^3$ represents a hydrogen atom and the other of $R^2$ and $R^3$ is the same as $R^1$, which comprises reacting a compound of general formula

$R^1-CH=C=CH_2$ (II)

with a salt of formic acid in the presence of a catalyst system comprising a palladium salt and a phosphorus compound of general formula

$P(OX)_n(X)_{3-n}$ (III)

in which n is 0,1,2 or 3 and each X independently represents an alkyl, alkenyl, aryl, aralkyl, alkaryl or cycloalkyl group, and recovering the desired compound of formula (I).

**2.** A process as claimed in claim 1, in which the salt of formic acid is an alkali metal salt or a salt of a tertiary organic amine.

**3.** A process as claimed in claim 1 or 2, in which the palladium salt is a carboxylate, nitrate or halide.

**4.** A process as claimed in claim 3, in which the palladium salt is palladium acetate.

**5.** A process as claimed in any one of claims 1 to 4, in which X represents an alkyl or alkenyl group having from 3 to 10 carbon atoms, a phenyl group, a di-tertiarybutylphenyl group or a cycloalkyl group having from 3 to 6 carbon atoms.

**6.** A process as claimed in claim 5, in which the phosphorus compound of general formula (III) is selected from triphenylphosphine, tri-isopropylphosphine, diphenylisopropylphosphine, tribenzylphosphine, tri-(ortho-toluyl)phosphine and tri-(2,4-di-tertiarybutylphenyl)phosphite.

**7.** A process as claimed in claim 6, in which the phosphorus compound of general formula (III), is tri-isopropylphosphine.

**8.** A process as claimed in any one of claims 1 to 7, in which the molar ratio of phosphorus compound of general formula (III) to palladium salt is in the range of from 1:1 to 20:1.

**9.** A process as claimed in any one of claims 1 to 8, in which the reaction is performed in the presence of a solvent selected from dimethylformamide, dimethylacetamide, pyridine, triethylamine, dimethylsulphoxide, tetrahydrofuran, benzonitrile, nitromethane, nitrobenzene and propylene carbonate.

**10.** A process as claimed in any one of claims 1 to 9, in which the pressure is in the range of from 2 to 25 bar.

## Patentansprüche

1. Verfahren zur herstellung einer Verbindung mit der allgemeinen Formel

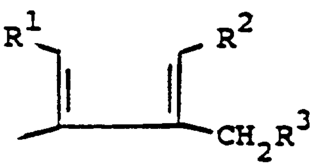

(I),

worin $R^1$ ein Wasserstoffatom oder eine Methylgruppe bedeutet, einer der Reste von $R^2$ und $R^3$ ein Wasserstoffatom darstellt und der andere Rest von $R^2$ und $R^3$ die gleiche Bedeutung wie $R^1$ aufweist, welches Verfahren ein Umsetzen einer Verbindung der allgemeinen Formel

$R^1 - CH = C = CH_2$ (II)

mit einem Salz der Ameisensäure in Anwesenheit eines ein Palladiumsalz und eine Phosphorverbindung der allgemeinen Formel

$P(OX)_n(X)_{3-n}$ (III)

umfassenden Katalysatorsystems, in welcher Formel n den Wert 0, 1, 2 oder 3 hat und jedes X unabhängig voneinander eine Alkyl-, Alkenyl-, Aryl-, Aralkyl-, Alkaryl- oder Cycloalkylgruppe bedeutet, und ein Gewinnen der gewünschten Verbindung der Formel (I) umfaßt.

2. Verfahren nach Anspruch 1, worin das Salz der Ameisensäure ein Alkalimetallsalz oder ein Salz eines tertiären organischen Amins ist.

3. Verfahren nach Anspruch 1 oder 2, worin das Palladiumsalz ein Carboxylat, Nitrat oder Halogenid ist.

4. Verfahren nach Anspruch 3, worin das Palladiumsalz Palladiumacetat ist.

5. Verfahren nach einem der Ansprüche 1 bis 4, worin eine Alkyl- oder Alkenylgruppe mit 3 bis 10 Kohlenstoffatomen, eine Phenylgruppe, eine Diterbutylphenylgruppe oder eine Cycloalkylgruppe mit 3 bis 6 Kohlenstoffatomen darstellt.

6. Verfahren nach Anspruch 5, worin die Phosphorverbindung der allgemeinen Formel (III) unter Triphenylphosphin, Triisopropylphosphin, Diphenylisopropylphoshin,Tribenzylphosphin, Tri(orthotoluyl)-phosphin und Tri(2,4-di-tertbutylphenyl)phosphit ausgewählt ist.

7. Verfahren nach Anspruch 6, worin die Phosphorverbindung der allgemeinen Formel (III) Triisopropylphosphin ist.

8. Verfahren nach einem der Ansprüche 1 bis 7, worin das Molverhältnis der Phosphorverbindung der allgemeinen Formel (III) zum Palladiumsalz im Bereich von 1:1 bis 20:1 liegt.

9. Verfahren nach einem der Ansprüche 1 bis 8, worin die Umsetzung in Anwesenheit eines unter Dimethylformamid, Dimethylacetamid, Pyridin, Triethylamin, Dimethylsulfoxid, Tetrahydrofuran, Benzonitril, Nitromethan, Nitrobenzol und Propylencarbonat ausgewählten Lösungsmittels ausgeführt wird.

10. Verfahren nach einem der Ansprüche 1 bis 9, worin der Druck im Bereich von 2 bis 25 bar liegt.

## Revendications

**1.** Un procédé pour la préparation d'un composé de formule générale:

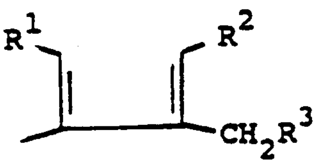

(I)

dans laquelle $R^1$ représente un atome d'hydrogène ou un groupe méthyle, l'un parmi $R^2$ et $R^3$ représente un atome d'hydrogène et l'autre parmi $R^2$ et $R^3$ est identique à $R^1$, procédé dans lequel on fait réagir un composé de formule générale

$R^1CH=C-CH_2$ (II)

avec un sel de l'acide formique en présence d'un système catalytique comportant un sel de palladium et un composé du phosphore de formule générale

$P(OX)_n(X)_{3-n}$(III)

dans laquelle n est 0,1,2 ou 3 et chaque X représente indépendamment un groupe alkyle, alcényle, aryle, aralkyle, alkaryle ou cycloalkyle, après quoi on recueille le composé souhaité de formule (I).

**2.** Un procédé tel que revendiqué dans la revendication 1, dans lequel le sel de l'acide formique est un sel de métal alcalin ou un sel d'une amine organique tertiaire.

**3.** Un procédé tel que revendiqué dans la revendication 1 ou 2, dans lequel le sel de palladium est un carboxylate, un nitrate ou un halogénure.

**4.** Un procédé tel que revendiqué dans la revendication 3, dans lequel le sel de palladium est l'acétate de palladium.

**5.** Un procédé tel que revendiqué dans l'une quelconque des revendications 1 à 4, dans lequel X représente un groupe alkyle ou alcényle possédant de 3 à 10 atomes de carbone, un groupe phényle, un groupe di-tert.butylphényle ou un groupe cycloalkyle possédant de 3 à 6 atomes de carbone.

**6.** Un procédé tel que revendiqué dans la revendication 5, dans lequel le composé du phosphore de formule générale (III) est choisi parmi la triphénylphosphine, la tri-isopropylphosphine, la diphénylisopropylphosphine, la tribenzylphosphine, la tri(orthotoluyl)phosphine et le tri(2,4-di-tert.butylphényl) phosphite.

**7.** Un procédé tel que revendiqué dans la revendication 6, dans lequel le composé du phosphore de formule générale (III), est la tri-isopropylphosphine.

**8.** Un procédé tel que revendiqué dans l'une quelconque des revendications 1 à 7, dans lequel le rapport molaire du composé du phosphore de formule générale (III) au sel de palladium se situe dans la gamme de 1:1 à 20:1.

**9.** Un procédé tel que revendiqué dans l'une quelconque des revendications 1 à 8, dans lequel la réaction est mise en oeuvre en présence d'un solvant choisi parmi le diméthylformamide, le diméthylacétamide, la pyridine, la triéthylamine, le diméthylsulfoxyde, le tétrahydrofurane, le benzonitrile, le nitrométhane, le nitrobenzène et le carbonate de propylène.

**10.** Un procédé tel que revendiqué dans l'une quelconque des revendications 1 à 9, dans lequel la pression se situe dans la gamme de 2 à 25 bars.